# EUROPÄISCHE PATENTSCHRIFT

(11) **EP 0 439 668 B1**
(45) Veröffentlichungstag und Bekanntmachung des Hinweises auf die Patenterteilung: **16.02.1994**
(21) Anmeldenummer: 90109563.8
(22) Anmeldetag: 19.05.1990
(51) Int. Cl.: A61L 17/00

(54) **Verfahren zum Behandeln eines chirurgischen Nähfadens und chirurgischer Nähfaden**
Method for treatment of a surgical filament and surgical filament
Procédé de traitement d'un filament chirurgical et filament chirurgical

(30) Priorität: 30.01.1990 DE 4002626
(43) Veröffentlichungstag der Anmeldung: 07.08.1991
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Planck, Heinrich, Dr.-Ing., D-7440 Nürtingen 10 (DE); Müller, Erhard, Dr. rer.nat., D-7000 Stuttgart 80 (DE)
(74) Vertreter: Patentanwälte Ruff, Beier, Schöndorf und Mütschele

(56) Entgegenhaltungen:
- EP-A- 0 170 966
- US-A- 4 201 216

## Beschreibung

Die Erfindung betrifft ein Verfahren zum Behandeln eines chirurgischen Nähfadens gemäß dem Oberbegriff des Anspruches 1 und einen nach diesem Verfahren hergestellten chirurgischen Nähfaden.

Es ist ein mehrfädiges Nahtmaterial bekannt, welches zur Verbesserung der Verknotungseigenschaften mit etwa 1 bis 5 Gewichtsprozent des trockenen Rückstandes einer Masse, die ein Gel eines Salzes einer Fettsäure mit 6 oder mehr Kohlenstoffatomen mit einem mehrwertigen Metallion in einem flüchtigen organischen Lösungsmittel enthält, überzogen ist (DE-OS 29 21 810). Bei diesem mehrfädigen Nahtmaterial handelt es sich um einen chirurgischen Nähfaden. Die Herstellung des der Beschichtung dienenden Gels ist jedoch zeitaufwendig und kostenaufwendig. Auch muß der Nähfaden beim Beschichten mit der Gelmasse an der Stelle seines Austritts aus der Gelmasse durch ein Polster aus weichem Filz hindurchgezogen werden, um überschüssige Beschichtungsmasse abzustreifen und einen möglichst gleichmäßigen Auftrag zu erhalten. Dieses Filzpolster kann sich jedoch relativ rasch ändern und garantiert keine gleichmäßige Beschichtung.

Ein ähnliches Nahtmaterial ist auch in US-A-4 201 216 beschrieben, wobei jedoch bei Raumtemperatur und in Auwesenheit eines filmbildenden Polymers eine Fettsäuresalzdispersion verarbeitet wird. In EP-A-0 170 966 wirddagegen das Fettsäuresalz in Pulverform eingesetzt.

Es ist deshalb eine Aufgabe der Erfindung, ein Verfahren der im Oberbegriff des Anspruches 1 genannten Art zum Behandeln eines chirurgischen Nähfadens zu schaffen, das ebenfalls die Verknotungseigenschaften des chirurgischen Nähfadens verbessert, sich jedoch zeitsparend und kostengünstiger durchführen läßt und gute Gleichmässigkeit der Beschichtung erreichen läßt.

Diese Aufgabe wird erfindungsgemäß durch das in Anspruch 1 angegebene Verfahren gelöst.

Bei diesem erfindungsgemäßen Verfahren findet das Beschichten des chirurgischen Nähfadens mittels einer Lösung statt, welche das Fettsäure-Metallsalz in einem Lösungsmittel gelöst enthält. Die Temperatur der Lösung beim Beschichten wird so hoch gehalten, daß das Fettsäure-Metallsalz gelöst bleibt und eine Gelbildung vermieden wird. Nach dem Beschichten des chirurgischen Nähfadens mittels der Lösung wird das Lösungsmittel aus der Beschichtung zu deren Trocknung bei so hoher Temperatur verdampft, daß das Beschichtungsmittel nach der Verdampfung auf dem Nähfaden als trockener Rückstand verbleibt.

Dieses erfindungsgemäße Verfahren ergibt Nähfäden mit ausgezeichneten Verknotungseigenschaften, und man kann dies wegen der gleichmäßigeren Verteilung des Fettsäure-Metallsalzes auch mit geringerem prozentualem Gewichtsanteil des Fettsäure-Metallsalzes an dem Gesamtgewicht des beschichteten Nähfadens als bei dem vorbekannten Beschichten des Nähfadens mit einem Gel desselben Fettsäure-Metallsalzes ereichen.

Das erfindungsgemäße Verfahren läßt sich ferner zeitsparend und sehr kostengünstig durchführen und ergibt problemlos eine sehr gleichmäßige Beschichtung. Zum Beschichten des Nähfadens genügt es, ihn bspw. durch die heiße Lösung nur hindurchzuziehen, was mit relativ hohen Vorschubgeschwindigkeiten stattfinden kann. Danach ist es lediglich noch erforderlich, den Nähfaden, d.h. seine Beschichtung, bei ausreichend hohen Temperaturen zu trocknen, was bspw. ebenfalls sehr rasch in einem beheizten Trocknungsraum oder Trocknungstunnel oder auch nur durch Anblasen mittels entsprechend heißer Luft oder sonstigem heißen Gas erfolgen kann. Hierzu kann der Nähfaden kontinuierlich durch den Trocknungsraum oder Trocknungstunnel oder den sonstigen Raum unmittelbar nach seinem Benetzen oder Tränken mit der Lösung hindurchgeführt werden. Bevorzugt erfolgt ein weiteres Trocknen unter vermindertem Druck, z. B. im Druckbereich von wenigen mbar, um letzte Lösungsmittelreste zu entfernen.

Das Aufbringen der Lösung auf den Nähfaden kann also vorzugsweise so erfolgen, daß der Faden durch die Lösung hindurchgezogen wird. Es ist dabei im allgemeinen danach nicht mehr erforderlich, den Faden noch durch Abstreifen an irgendeinem Abstreifer von der Lösung vor deren Trocknen teilweise wieder zu befreien, so daß im allgemeinen nach dem Herausziehen des Fadens aus der Lösung sofort anschließend ohne Zwischenbehandlung mit dem Trocknen der an ihm anhaftenden Lösung begonnen werden kann.

Das Aufbringen der Lösung auf den chirurgischen Nähfaden kann auch noch auf andere Weise erfolgen, bspw. durch Anspritzen oder Besprühen mit der heißen Lösung, oder durch Auftrag mittels einer oder mehreren Auftragswalzen, oder auf sonstige Weise.

Das Fettsäure-Metallsalz kann oft besonders vorteilhaft aus einer einzigen chemischen Verbindung bestehen. Jedoch kann als Fettsäure-Metallsalz auch ein Gemisch aus Salzen mehrerer Fettsäuren verwendet werden.

Besonders vorteilhaft ist es, vorzusehen, daß als Fettsäure-Metallsalz ein Magnesium-Fettsäuresalz, vorzugsweise Magnesiumstearat, und/oder ein Bariumsalz und/oder ein Aluminiumsalz und/oder ein Zinksalz und/oder ein Calciumsalz, vorzugsweise Calciumstearat, verwendet wird.

Als Lösungsmittel wird bevorzugt ein Terpengemisch mit Limonen als Hauptbestandteil verwendet, das von der Firma Carl Roth GmbH & Co., Karlsruhe, unter dem Namen Rotihistol vertrieben wird. Es kommen auch andere geeignete Lösungsmittel infrage, z. B. aromatische Kohlenwasserstoffe, wie Benzol, Toluol, Xylol, Mesitylen.

Bei einer bevorzugten Ausführungsform ist vorgesehen, daß in der Lösung auch mindestens ein Wachs gelöst oder dispergiert ist, das sich so beim Tränken oder Benetzen des Nähfadens mit der Lösung zusätzlich zu dem Fettsäure-Metallsalz auf dem Nähfaden mit niederschlägt und die Verknotungseigenschaften weiter verbessert und/oder die Haftung des Fettsäure-Metallsalzes am Nähfaden verbessert, indem es in die Beschichtung des Nähfadens als zusätzlicher Bestandteil mit eingeht. Der Gewichtsanteil des Wachses bezogen auf das Gesamtgewicht der Beschichtung des Nähfadens kann vorzugsweise 10 bis 90 Gew.%, besonders zweckmäßig 20 bis 50 Gew.% betragen. Das Wachs kann bspw. eine Mischung nichtionogener Ester sein, z.B. Sorbitanmonostearat. Vorzugsweise kann es ein nicht polymeres Wachs sein.

Die Temperatur der Lösung beim Beschichten des Nähfadens ist zumindest so hoch zu wählen, daß das Fettsäure-Metallsalz im Lösungsmittel gelöst bleibt. Das Trocknen des Nähfadens nach dem Tränken oder Benetzen mit der Lösung wird ebenfalls bei hohen Temperaturen durchgeführt, um gute Verknotungseigenschaften zu erhalten.

Die minimale Temperatur der Lösung beim Beschichten des Nähfadens, d.h. bei dessen Tränken oder Benetzen mit der Lösung hat sich mit nach dem Fettsäure-Metallsalz zu richten und kann in vielen Fällen, beispielsweise bei Magnesiumstearat als Fettsäure-Metallsalz, vorzugsweise mindestens 50° C betragen und je nach Fettsäure-Metallsalz auch höher oder niedriger sein.

Das Trocknen des Nähfadens kann in Gasatmosphäre, vorzugsweise in Luft erfolgen, wobei sich die minimale Temperatur des Gases ebenfalls mit nach dem Fettsäure-Metallsalz und nach dem verwendeten Lösungsmittel zu richten hat und in vielen Fällen, bspw. bei Verwendung von Magnesiumstearat als Fettsäure-Metallsalz, vorzugsweise mindestens 80° C und max. 200° C betragen kann.

Die Erfindung erstreckt sich auch auf einen chirurgischen Nähfaden, welcher mit einer erfindungsgemäßen Beschichtung versehen ist. Bevorzugt kann dabei vorgesehen sein, daß die Beschichtung ca. 0,5 bis 10 Gew.% des beschichteten Nähfadens beträgt.

In der Zeichnung sind Ausführungsbeispiele der Erfindung dargestellt. Es zeigen
- Fig. 1 und 2: je eine Einrichtung zum Behandeln von chirurgischen Nähfäden gemäß Ausführungsbeispielen der Erfindung in im wesentlichen schematischer und teilweise geschnittener und gebrochener Darstellung,
- Fig. 3: eine Draufsicht auf den Avivierstift der Einrichtung nach Fig. 2 in vergrösserter Darstellung,
- Fig. 4: einen Schnitt durch den Avivierstift nach Fig. 3, gesehen entlang der Schnittlinie 4-4.

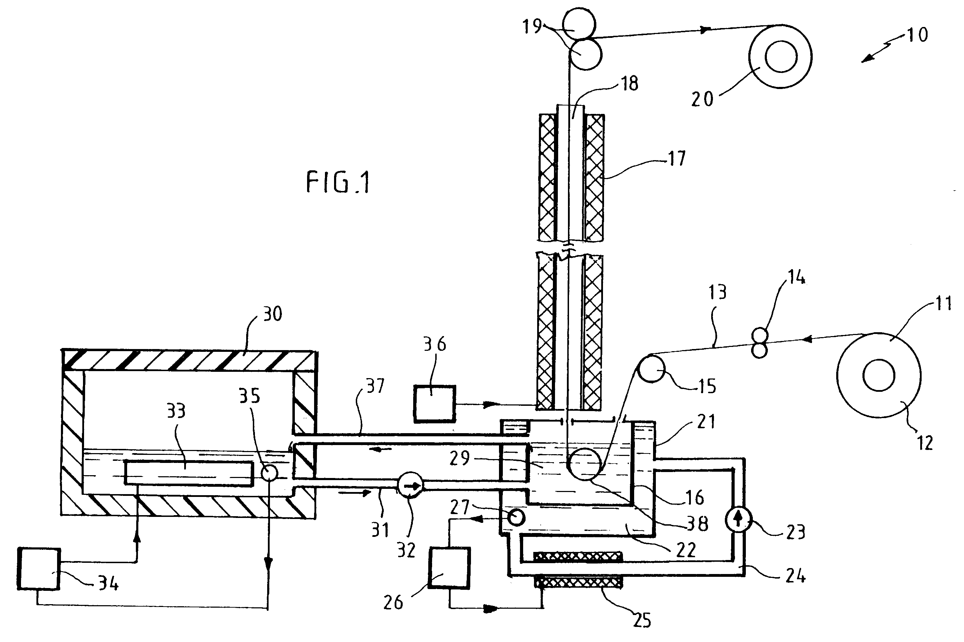

Die Einrichtung 10 nach Fig. 1 weist eine Abwickelspule 11 auf, deren Fadenwicklungskörper 12 aus einem zu beschichtenden chirurgischen Nähfaden besteht. Dieser chirurgische Nähfaden 13 wird von dieser Abwickelspule 12 im Betrieb der Einrichtung 10 stetig abgewickelt und durchläuft dann zuerst eine seinem Spannen dienende Fadenbremse 14 und läuft von dieser zu einer Umlenkrolle 15 und dann weiter zu einer in einem Tränkgefäß 16 angeordneten weiteren Umlenkrolle 38 und von dieser aus vertikal nach oben durch einen mittels einer Heizwicklung 17 beheizten Trocknungskanal 18 zu einem oberhalb des Trocknungskanals 18 angeordneten angetriebenen Walzenpaar 19, welches den durch die Fadenbremse 14 mit vorbestimmter Spannung gespannten Nähfaden 13 mit konstanter Vorschubgeschwindigkeit von bspw. 3 bis 20 m/s zu einer ihn aufwindenden, angetriebenen Aufwickelspule 20 liefert.

Das Tränkgefäß 16 befindet sich innerhalb eines Heizgefäßes 21, in welchem sich ein flüssiges Heizmedium 22, bspw. Öl mit einem Siedepunkt von über 200° C befindet, welches im Betrieb dieser Einrichtung 10 mittels einer Pumpe 23, die sich in einer vom Boden des Heizgefäßes 21 bis zu einer Seitenwand des Heizgefäßes 21 führenden Umgehungsleitung 24 befindet fortlaufend aus dem Heizgefäß 21 abgesaugt und an einer weiter oben gelegenen Stelle wieder in dieses zurückgeleitet wird. Diese Umgehungsleitung 24 weist eine Heizwicklung 25 auf, die dem Beheizen des Heizmediums 22 auf eine konstante Temperatur mittels eines Temperaturreglers 26 dient, dem der Ist-Wert der Temperatur des Heizmediums 21 durch einen innerhalb des Heizgefäßes 21 angeordneten Temperaturfühler 27 eingegeben wird. Der Temperaturregler 26 vergleicht diesen Ist-Wert mit einem an ihm eingestellten Soll-Wert der Temperatur des Heizmediums 22 und steuert die der Heizwicklung 25 zugeführte Energie in der Weise, daß die vom Fühler 27 gemessene Temperatur des Heizmediums innerhalb des Heizgefäßes 22 ungefähr auf dem vorbestimmten Soll-Wert gehalten wird.

In dem Tränkgefäß 16 befindet sich eine Lösung 29, mit der der chirurgische Nähfaden 13 getränkt und damit beschichtet wird. Es handelt sich hier um eine Lösung, die aus einem Lösungsmittel und einem in diesem gelösten Fettsäure-Metallsalz mit sechs oder mehr Kohlenstoffatomen besteht. Nachfolgend werden Beispiele hierfür angegeben. Bevorzugt enthält die Lösung ein aus Fettsäureestern bestehendes Wachs.

Die im Tränkgefäß 16 angeordnete Umlenkrolle 18 ist in die im Tränkgefäß 16 befindliche Lösung 29 eingetaucht und bewirkt so das Eintauchen des Fadens 13 in diese Lösung 29.

Diese Lösung 29 ist nicht nur im Tränkgefäß 16 vorhanden, sondern auch in einem mit ihm über Leitungen 31 und 37 ständig verbundenen Vorratsgefäß 30.

Im Betrieb wird die im Vorratsgefäß 30 befindliche Lösung 29 fortlaufend mittels der das Vorratsgefäß 30 nahe an seinem Boden mit dem Tränkgefäß 16 nahe an dessen Boden verbindenden Verbindungsleitung 31 mit in diese eingesetzter Pumpe 32 fortlaufend in das Tränkgefäß 16 gepumpt und es strömt aus diesem nahe dessen Oberseite über die ungefähr horizontale Überlaufleitung 37 in das Vorratsgefäß 30 zurück.

Diese Lösung 29 wird im Tränkgefäß 16 durch das Heizmedium 22 und im Vorratsgefäß 30 durch eine elektrische Heizung 33 beheizt, damit sie im Tränkgefäß 16 eine einstellbare, vorbestimmte hohe Temperatur einhält, bei der das Fettsäure-Metallsalz im Lösungsmittel der Lösung 29 sicher vollständig gelöst ist, also nicht geliert und nicht dispergiert ist.

Die Heizung 33 ist an einen Temperaturregler 34 angeschlossen, an den ein innerhalb der Lösung 29 im Vorratsgefäß 30 befindlicher Temperaturfühler 35 als Ist-Wert-Fühler angeschlossen ist. Dieser Temperaturfühler 35 meldet den Ist-Wert der Temperatur der Lösung 29 im Vorratsgefäß 30 dem Regler 34, welcher diesen mit einem an ihm eingestellten Soll-Wert vergleicht, der dem am Temperaturregler 26 eingestellten Temperatur-Soll-Wert entspricht oder gringfügig grösser ist, und der Regler 34 steuert die Heizung 33 so an, daß die Temperatur der Lösung 29 im Vorratsgefäß 30 ständig ungefähr dem am Temperaturregler 34 eingestellten Soll-Wert dieser Temperatur entspricht.

Die Heizwicklung 17 des Trocknungskanals 18 wird mittels einer Temperatursteuervorrichtung 36 mit Heizstrom beschickt, wobei ggfs. auch eine Temperaturregelung der Temperatur der Heizwicklung 17 vorgesehen sein kann. Die Temperatur im Trocknungskanal 18 kann zweckmäßig ungefähr gleich groß oder größer als die Temperatur der Lösung 29 sein, je nach der Höhe der Temperatur der Lösung 29 jedoch auch etwas niedriger.

Wesentlich ist, daß die Temperatur der Lösung 29 und die dem Trocknen des vorangehend in dem Tränkgefäß mit der Lösung 29 getränkten Nähfadens 13 dienende Lufttemperatur im Trocknungskanal 18 so hoch eingestellt sind, daß die Kombination von Fettsäure-Metallsalz und Wachs in der den Nähfaden 13 benetzenden Lösung 29 auch während des Trocknens des Nähfadens 13 im Trocknungskanal 18 gelöst bleibt, also bis das Lösungsmittel, welches der Nähfaden 13 zusammen mit dem in ihm gelösten Fettsäure-Metallsalz und dem in ihm gelösten Wachs im Tränkgefäß 16 aufnahm, verdampft ist, so daß dann am Nähfaden 13 nur das Fettsäure-Metallsalz und das Wachs zurückgeblieben ist und dessen Knotenlaufeigenschaften beträchtlich verbessert. Der Trockungskanal 18 ist also mit so hoher Temperatur beheizt, daß das Lösungsmittel in dem Trocknungskanal 18 durch die hohe Temperatur in diesem vollständig vom Nähfaden 13 abgedampft wird. Der Nähfaden 13 verläßt also den Trocknungskanal 18 in getrocknetem Zustand, so daß nunmehr der Nähfaden 13 nur noch mit dem Fettsäure-Metallsalz und dem Wachs als Feststoff beschichtet den Trocknungskanal 18 verläßt. Hierdurch ist die Verteilung von Fettsäure-Metallsalz und Wachs auf dem chirurgischen Nähfaden besonders gleichmäßig, und es werden besonders gute Verknotungseigenschaften des chirurgischen Nähfadens erzielt. Auch kommt man mit sehr kleinen Mengen an Fettsäure-Metallsalz aus.

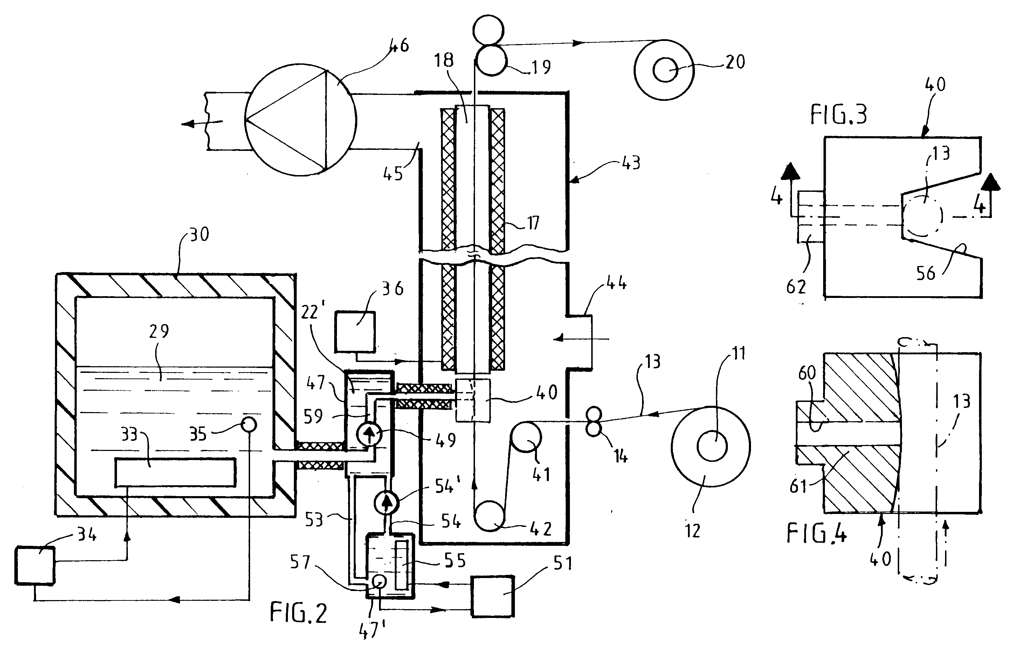

Bei der Einrichtung 10' nach Fig. 2 erfolgt das Beschichten des mittels eines angetriebenen Walzenpaares 19 von einer Abwickelspule 11 zu einer Aufwickelspule 20 mit konstanter Vorschubgeschwindigkeit geförderten chirurgischen Nähfadens 13 mittels eines sogenannten Avivierstiftes 40, dessen Bauart näher in Fig. 3 und 4 dargestellt ist und weiter unten erläutert wird.

In geringem Abstand vertikal oberhalb des Avivierstiftes 40 befindet sich wiederum ein Trocknungskanal 18 mit einer Heizwicklung 17, welche letztere durch eine Temperatursteuervorrichtung 36 wie bei der Einrichtung 10 nach Fig. 1 mit Heizstrom beschickt wird. Mit 14 ist wiederum eine den Nähfaden 13 zwischen sich und dem Walzenpaar 19 spannende Fadenbremse bezeichnet, und ferner wird der chirurgische Nähfaden 13 nach der Fadenbremse 14 über eine Umlenkrolle 41 zu einer unteren Umlenkrolle 42 und von dieser vertikal aufwärts zum Avivierstift 40 und von diesem aus vertikal durch den Trocknungskanal 18 hindurch zu dem Antriebswalzenpaar 19 geleitet, das ihn zu der angetriebenen Aufwickelspule 20 weiterleitet.

Der Avivierstift 40, die Umlenkwalzen 41 und 42 und der Trocknungskanal 18 mit Heizwicklung 17 befinden sich innerhalb einer durch die Heizwicklung 17 mitbeheizten Heizkammer 43, welche seitlich einen einzigen Lufteinlaß 44 und auf ihrer dem Lufteinlaß 44 gegenüberliegenden Seite nahe ihrer Oberseite einen einzigen Luftauslaß 45 aufweist, der an eine Luftfördervorrichtung, bspw. einen Ventilator 46, angeschlossen ist, welcher dem Hindurchsaugen von Luft durch die Heizkammer 43 im Betrieb dient.

In einem Vorratsbehälter 30 befindet sich eine Lösung 29 von in einem Lösemittel gelösten Wachs und Fettsäure-Metallsalz, welches Fettsäure-Metallsalz sechs oder mehr Kohlenstoffatome aufweist und in dieser Lösung gelöst gehalten wird, indem die Temperatur dieser Lösung auf eine das Fettsäure-Metallsalz gelöst haltende hohe Temperatur mittels eines Temperaturreglers 34 geregelt wird. Diesem Temperaturregler 34 ist wie bei der Einrichtung 10 nach Fig. 1 eine von ihm angesteuerte, innerhalb der Lösung 29 angeordnete elektrische Heizung 33 und ein Ist-Wert-Temperaturfühler 35 innerhalb der Lösung 29 zugeordnet. Die Temperatur der Lösung 29 im Vorratsgefäß 30 wird so auf einem am Temperaturregler 34 eingestellten hohen Soll-Wert gehalten, bei welchem das Wachs und das Fettsäure-Metallsalz in der Lösung 29 gelöst bleiben.

Innerhalb eines Behälters 47 ist eine Dosierpumpe 49 in einer vom Vorratsgefäß 30 zum Avivierstift 40 führenden Leitung 59 für die heiße Lösung 29 angeordnet, um diese heiße Lösung 29 in vorbestimmter Menge/Zeit, d.h. dosiert, zum Avivierstift 40 zu fördern. Der Behälter 47 ist mit einem flüssigen Heizmedium 22' im wesentlichen gefüllt, dessen Temperatur mittels eines Temperaturreglers 51 auf einer hohen Temperatur gehalten wird, deren Soll-Wert dem Soll-Wert der Temperatur der Lösung 29 im Vorratsgefäß 30 ungefähr entspricht. Dieser Behälter 47 ist mit einem Heizbehälter 47' über eine Hinleitung 53 und eine Rückleitung 54 verbunden. In der Rückleitung 54 ist eine Pumpe 54' angeordnet, die das in den Behältern 47 und 47' befindliche flüssige Heizmedium 22' ständig umwälzt. An den Temperaturregler 51 ist als Ist-Wert-Fühler ein im Behälter 47' angeordneter Temperaturfühler 57 angeschlossen, und der Temperaturregler 51 steuert eine in dem Behälter 47' angeordnete elektrische Heizung 55 zur Konstanthaltung der hohen Temperatur des Heizmediums 22' an. Hierdurch wird die Dosierpumpe 49 auf der hohen Temperatur der sie durchströmenden Lösung 29 gehalten. Die Verbindungsleitung 59 ist zwischen dem Vorratsgefäß 30 und dem Behälter 47 wie auch zwischen diesem Behälter 47 und dem Avivierstift 40 durch Wärmeisolierungen gut wärmeisoliert, so daß der Avivierstift 40 die von der Dosierpumpe 49 geförderte Lösung 29 mit der am Temperaturregler 34 eingestellten hohen Temperatur empfängt und auf den chirurgischen Nähfaden 13 zu dessen Beschichtung aufbringt.

In der Heizkammer 43 wird durch die Heizung 17 eine hohe Temperatur aufrechterhalten, die ebenfalls ungefähr der Temperatur der Lösung 29 im Vorratsgefäß 30 entspricht oder höher ist oder in manchen Fällen auch etwas kleiner sein kann. Wesentlich ist, daß die Temperatur am Avivierstift 40 und auch in dem ihm nachgeordneten Trocknungskanal 18, wo das Lösungsmittel der mittels des Avivierstiftes 40 auf den Nähfaden 13 zu dessen Beschichtung gleichmässig aufgebrachten Lösung 29 vom Nähfaden 13 verdampft wird, so hoch ist, daß das Lösungsmittel der Lösung 29 entfernt wird und das Wachs und das Fettsäure-Metallsalz auf dem Faden 13 als trockener Rückstand verbleiben. Wachs und Fettsäure-Metallsalz behalten dann diese Konsistenz bei und verbessern so die Verknotungseigenschaften des Nähfadens 13 entsprechend.

Die Vorschubgeschwindigkeit des Nähfadens 13 kann wiederum bspw. zweckmäßig 3 bis 20 m/s betragen, je nach Sachlage auch noch größer oder auch kleiner sein, was auch auf die Einrichtung nach Fig. 1 entsprechend zutrifft.

Der Avivierstift 40 ist ein massiver Körper aus Metall oder Keramik, der einen im Querschnitt ungefähr trapezförmigen, jedoch an seiner Breitseite offenen Kanal 56 aufweist, an dessen Boden der in den Fig. 3 und 4 strichpunktiert angedeutete chirurgische Nähfaden 13, wie hier dargestellt, anliegt, wobei dieser Boden in der Schnittdarstellung nach Fig. 4 konvex, vorzugsweise kreisbogenförmig gewölbt ist. Der vertikal laufende Faden 13 läuft dabei über die Austrittsmündung eines den Bereich 61 und einen Anschlußstutzen 62 des Avivierstiftes 40 durchdringenden Kanals 60 für die Lösung 29, welche in dosierter Menge durch die Dosierpumpe 49 in den Kanal 60 und damit zum Faden 13 zu dessen gleichmäßiger Beschichtung gefördert wird. Unmittelbar nach dem Auftrag der Lösung auf den Nähfaden 13 läuft dieser vom Avivierstift 40 in den Trocknungskanal 18 ein und durchläuft diesen, ohne dessen Innenwandung zu berühren, zum angetriebenen Walzenpaar 19, wobei er auf dem Weg innerhalb des Trocknungskanals 18 vollständig getrocknet wird, indem in dem Kanal 18 das Lösungsmittel der auf den Faden 13 aufgebrachten Lösung verdampft wird.

Die nachfolgenden Beispiele erläutern die Erfindung.

### Beispiel 1.

300 g Calciumstearat und 100 g Wachs G1564 (Atlas-Chemie, Essen; Gemisch von Fettsäureestern) werden zu 4000 g Rotihistol (Terpengemisch) zugegeben und bei ca. 130° C binnen drei Stunden unter Rühren gelöst. Die heiße Lösung wird in einen auf 130° C beheizten Vorratsbehälter 30 (Fig. 1) gegeben und durch einen ebenfalls auf 130° C temperierten, kleineren Behälter 16 im Kreislauf gepumpt, vgl. Fig. 1.
Ein geflochtener Nähfaden der Stärke -3/0- aus Polyethylenterephetalat wird durch die heiße Lösung im Behälter 16 geleitet und mit dieser Lösung beschichtet. Unmittelbar danach wird der Nähfaden durch einen Heizkanal 17, der auf 160° C temperiert ist, geführt, wobei das Lösungsmittel abdampft. Der verbleibende Rest an Lösungsmittel wird bei 50° C und einem Unterdruck (Vakuum) von 1 mbar entfernt. Auf dem homogen beschichteten Faden verbleiben ca. 3 Gew. % fester Bestandteile, bestehend aus dem Wachs G1564 und Calciumstearat.

Der Nähfaden besitzt hervorragende Knotenlaufeigenschaften sowohl im trockenen wie auch im feuchten Zustand. Ein Nähfaden der Stärke -0- zeigt vergleichbare Eigenschaften.

### Beispiel 2.

300 g Magnesiumstearat und 100 g Wachs G1564 werden in 4000 g Xylol (Isomerengemisch) bei ca. 120° C binnen drei Stunden unter Rühren gelöst. Die heiße Lösung wird in einen auf 60° C beheizten Vorratsbehälter 30 (Fig. 1) gegeben und durch einen ebenfalls auf 60° C temperierten kleineren Behälter 16 im Kreislauf gepumpt. Ein absorbierbarer, geflochtener Nähfaden der Stärke -3/0- aus Poly-(p-dioxanon) wird durch die heiße Lösung im Behälter 16 geleitet und mit dieser Lösung beschichtet. Unmittelbar danach wird der Nähfaden durch einen Heizkanal 17, der auf 140° C temperiert ist, geführt, wobei das Lösungsmittel abdampft. Der verbleibende Rest an Lösungsmittel wird bei 50° C unter Lagerung bei einem Unterdruck (Vakuum) von 1 mbar entfernt. Auf dem homogen beschichteten Faden verbleiben ca. 3 Gew. % fester Bestandteile, bestehend aus dem Wachs G1564 und Calciumstearat. Der Nähfaden besitzt hervorragende Knotenlaufeigenschaften sowohl im trockenen wie im feuchten Zustand.

### Beispiel 3.

Entsprechend den allgemeinen Verfahren von Beispiel 1 und 2 werden unter Verwendung von Benzol, Toluol, Xylol, Mesitylen und Rotihistol als organische Lösungsmittel mit Calciumstearat und dem Wachs G1564 Nahtmaterialien aus heißer Lösung homogen beschichtet. Statt Calciumstearat kann ebenso Magnesium- bzw. Zinkstearat verwendet werden, die in Kombination mit dem Wachs G1564 ähnlich verbesserte Verknotungseigenschaften zeigen wie die Kombination von Calciumstearat und dem Wachs G1564.

### Beispiel 4.

Eine Lösung aus 100 g Wachs G1564, 500 g Calciumstearat und 4000 g Rotihistol wird bei 120° C mittels eines Avivierstiftes auf einen Nähfaden der Starke -3/0-, bestehend aus Poly-(p-dioxanon) aufgebracht. Die Lösung befindet sich hierbei in einem auf 120° C temperierten Vorratsbehälter 30 und wird mittels einer 120° C warmen Dosierpumpe 49 durch ein beheiztes Kupferrohr zum ebenfalls auf 120°C beheizten Avivierstift 40 befördert, vgl. die Figuren 2 bis 4. Der Faden wird an der Austrittsöffnung 60 des Avivierstiftes 40 vorbeigeführt und wird gleichmäßig mit der Lösung beschichtet. Unmittelbar danach wird der Faden durch einen 140° C heißen Heizkanal 17 geleitet und weitgehend vcm Lösungsmittel befreit. Der verbleibende Rest an Lösungsmittel wird bei 50° C unter Lagerung des Fadens bei einem Unterdruck (Vakuum) von 1 mbar entfernt.

Auf dem homogen beschichteten Nähfaden verbleiben ca. 5 Gew.% fester Bestandteile, bestehend aus dem Wachs G1564 und Calciumstearat. Der Nähfaden besitzt hervorragende Knotenlaufeigenschaften sowohl im trockenen wie im feuchten Zustand.

### Beispiel 5.

Entsprechend dem Verfahren nach Beispiel 4 werden unter Verwendung von Benzol, Toluol, Xylol, Mesitylen und Rotihistol als organische Lösungsmittel mit Wachs G1564 und Calciumstearat Nahtmaterialien aus heißer Lösung mittels eines Avivierstiftes beschichtet. Statt Calciumstearat kann ebenso Magnesium- bzw. Zinkstearat verwendet werden, die in Kombination mit dem Wachs G1564 ähnlich verbesserte Verknotungseigenschaften zeigen wie die Kombination des Wachses G1564 mit Calciumstearat. Da Zink für die Wundheilung sehr wichtig ist, kann die Verwendung von Zinkstearat pharmakologisch besonders günstig sein.

Die Beschichtung kann außer den erläuterten Bestandteilen auch solche Bestandteile enthalten, die für den Verwendungszweck erforderlich sind, z.B. Antibiotika, Antiseptika, Antiphlogistika, Farbstoffe etc.

Die Geschwindigkeit des chirurgischen Fadens bei der Beschichtung betrug bei allen Beispielen 8 m/min.

## Patentansprüche

1. Verfahren zum Behandeln eines chirurgischen Nähfadens, der aus einer Mehrzahl von Filamenten besteht und mit einer Beschichtung versehen wird, die ein Metallsalz einer Fettsäure mit 6 oder mehr Kohlenstoffatomen aufweist,
dadurch gekennzeichnet, daß das Fettsäure-Metallsalz in einem Lösungsmittel gelöst wird,
daß der Nähfaden mit dieser Lösung beschichtet wird, wobei ihre Temperatur so hoch gehalten wird, daß das Fettsäure-Metallsalz gelöst bleibt und eine Gelbildung vermieden wird und daß danach das Lösungsmittel aus der auf den Nähfaden aufgebrachten Beschichtung zu deren Trocknung bei erhöhter Temperatur verdampft wird.

2. Verfahren nach Anspruch 1, dadurch gekennzeichnet daß in der Lösung vor dem Beschichten des Nähfadens auch mindestens ein Wachs gelöst wird.

3. Verfahren nach Anspruch 1 oder 2, dadurch gekennzeichnet, daß der Nähfaden zur Beschichtung durch die Lösung hindurchgeführt wird.

4. Verfahren nach einem der vorhergehenden Ansprüche, dadurch gekeinnzeichnet, daß die Temperatur der Lösung beim Beschichten des Nähfadens mindestens 50° C beträgt.

5. Verfahren nach einem der vorhergehenden Ansprüche, dadurch gekennzeichnet, daß das Trocknen des Nähfadens in Gasatmosphäre bei einer Gastemperatur von mindestens 80° C und höchstens 200° C durchgeführt wird.

6. Verfahren nach einem der vorhergehenden Ansprüche, dadurch gekennzeichnet, daß als Fettsäure-Metallsalz ein aus einer einzigen chemischen Verbindung bestehendes Fettsäure-Metallsalz, vorzugsweise Magnesiumstearat, Calciumstearat oder Zinkstearat, verwendet wird.

7. Verfahren nach einem der Ansprüche 1 bis 5, dadurch gekennzeichnet, daß als Fettsäure-Metallsalz ein Gemisch aus Salzen mehrerer Fettsäuren verwendet wird.

8. Verfahren nach einem der vorhergehenden Ansprüche, dadurch gekennzeichnet, daß als Fettsäure-Metallsalz ein Magnesiumsalz und/oder ein Bariumsalz und/oder ein Aluminiumsalz und/oder ein Zinksalz und/oder ein Calciumsalz verwendet wird.

9. Verfahren nach einem der vorhergehenden Ansprüche, dadurch gekennzeichnet, daß als organisches Lösungsmittel ein Terpengemisch verwendet wird.

10. Verfahren, nach einem der Ansprüche 1 bis 8, dadurch gekennzeichnet, daß als organisches Lösungsmittel eines aus der Gruppe Benzol, Toluol, Xylol und Mesitylen verwendet wird.

11. Chirurgischer Nähfaden, erhältlich mit einem Verfahren nach einem der vorhergehenden Ansprüche.

12. Chirurgischer Nähfaden nach Anspruch 11, dadurch gekennzeichnet, daß die Beschichtung ca. 0,5 bis 10 Gew.% des beschichteten Nähfadens beträgt.

13. Chirurgischer Nähfaden nach Anspruch 11 oder 12, dadurch gekennzeichnet, daß der Gewichtsanteil des Wachses, bezogen auf das Gesamtgewicht der Beschichtung des Nähfadens, 10 bis 90 % und bevorzugt 20 bis 50 % beträgt.

## Claims

1. Process for the treatment of a surgical suturing thread, which comprises a plurality of filaments and which is given a coating, which contains a metal salt of a fatty acid with 6 or more carbon atoms, characterized in that the fatty acid metal salt is dissolved in a solvent, that the suturing thread is coated with this solution, its temperature being kept so high that the fatty acid metal salt remains in a dissolved state and gel formation is avoided and that subsequently the solvent is evaporated from the coating applied to the suturing thread for its drying at elevated temperature.

2. process according to claim 1, characterized in that at least one wax is dissolved in the solution prior to the coating of the suturing thread.

3. process according to claim 1 or 2, characterized in that, for coating purposes, the suturing thread is passed through the solution.

4. process according to any one of the preceding claims, characterized in that the temperature of the solution on coating the suturing thread is at least 50°C.

5. Process according to any one of the preceding claims, characterized in that the suturing thread is dried in a gaseous atmosphere at a gas temperature of min 80°C and max 200°C.

6. Process according to any one of the preceding claims, characterized in that the fatty acid metal salt comprises a single chemical compound, preferably magnesium stearate, calcium stearate or zinc stearate.

7. process according to any one of the claims 1 to 5, characterized in that the fatty acid metal salt is constituted by a mixture of salts of several fatty acids.

8. Process according to any one of the preceding claims, characterized in that the fatty acid metal salt is constituted by a magnesium salt and/or a barium salt and/or an aluminium salt and/or a zinc salt and/or a calcium salt.

9. Process according to any one of the preceding claims, characterized in that a terpene mixture is used as the organic solvent.

10. Process according to any one of the claims 1 to 8, characterized in that the organic solvent is chosen from the group benzene, toluene, xylene and mesitylene.

11. Surgical suturing thread obtainable by a process according to one of the preceding claims.

12. Surgical suturing thread according to claim 11, characterized in that the coating represents approximately 0.5 to 10% by weight of the coated suturing thread.

13. Surgical suturing thread according to claim 11 or 12, characterized in that the weight proportion of the wax, based on the total weight of the coating of the suturing thread is 10 to 90%and preferably 20 to 50%.

## Revendications

1. Procédé de traitement d'un fil à suturer chirurgical, constitué de plusieurs filaments et pourvu d'une enduction composée d'un sel métallique d'acide gras à 6 atomes de carbone au moins, caractérisé en ce que le sel métallique d'acide gras est dissous dans un solvant, que le fil à suturer est enduit de cette solution, la température de celle-ci étant maintenue à une valeur telle que le sel métallique d'acide gras reste dissous et qu'il ne puisse se former de gel, et que le solvant est ensuite évaporé de l'enduction appliquée sur le fil à suturer lors d'un séchage à haute température.

2. Procédé selon la revendication 1, caractérisé en ce qu'une cire au moins est également dissoute dans la solution d'enduction du fil à suturer.

3. Procédé selon la revendication 1 ou 2, caractérisé en ce que le fil à suturer est amené à traverser la solution en vue de son enduction.

4. Procédé selon l'une des revendications précédentes, caractérisé en ce que la température de la solution lors de l'enduction du fil à suturer est de 50°C au moins.

5. Procédé selon l'une des revendications ci-dessus, caractérisé en ce que le séchage du fil à suturer en atmosphère gazeuse se fait à une température du gaz de 80°C au moins et 200°C au maximum.

6. Procédé selon l'une des revendications précédentes, caractérisé en ce que le sel métallique d'acide gras utilisé est constitué d'un seul composé chimique, de préférence du stéarate de magnésium, du stéarate de calcium ou du stéarate de zinc.

7. Procédé selon l'une des revendications 1 à 5, caractérisé en ce que le sel métallique d'acide gras utilisé est un mélange de sels de différents acides gras.

8. Procédé selon l'une des revendications précédentes, caractérisé en ce que le sel métallique d'acide gras utilisé est un sel de magnésium et/ou un sel de baryum et/ou un sel d'aluminium et/ou un sel de Zinc et/ou un sel de calcium.

9. Procédé selon l'une des revendications précédentes, caractérisé en ce que le solvant organique utilisé est un mélange de terpènes.

10. Procédé selon l'une des revendications 1 à 8, caractérisé en ce que le solvant organique utilisé est un solvant du groupe benzène, toluène, xylène et mésitylène.

11. Fil à suturer chirurgical, préparé par un procédé selon l'une des revendications précédentes.

12. Fil à suturer chirurgical selon la revendication 11, caractérisé en ce que l'enduction correspond à environ 0,5 à 10% en poids du fil enduit.

13. Fil à suturer chirurgical selon la revendication 11 ou 12, caractérisé en ce que la fraction pondérale de la cire, par rapport au poids total de l'enduction du fil à suturer, est de 10 à 90% et, de préférence, de 20 à 50%.
